# EUROPÄISCHE PATENTSCHRIFT

(11) **EP 1 955 062 B1**
(45) Veröffentlichungstag und Bekanntmachung des Hinweises auf die Patenterteilung: **22.12.2010**
(21) Anmeldenummer: 06791625.4
(22) Anmeldetag: 23.08.2006
(51) Int. Cl.: G01N 33/28

(54) **VORRICHTUNG ZUM ERMITTELN DER FILTRIERBARKEIT VON FLUIDEN, INSBESONDERE VON GETRIEBEÖLEN**
APPARATUS FOR DETERMINING THE FILTERABILITY OF FLUIDS, IN PARTICULAR OF TRANSMISSION OILS
DISPOSITIF DE DETERMINATION DE LA FILTRABILITE DE FLUIDES ET EN PARTICULIER D'HUILES DE TRANSMISSION

(30) Priorität: 07.11.2005 DE 102005053417
(43) Veröffentlichungstag der Anmeldung: 13.08.2008
(73) Patentinhaber: HYDAC FILTERTECHNIK GMBH, 66280 Sulzbach/Saar (DE)
(72) Erfinder: SCHMIDT, Armin, 66557 Illingen (DE)
(74) Vertreter: Bartels & Partner
(86) Internationale Anmeldenummer: PCT/EP2006/008265
(87) Internationale Veröffentlichungsnummer: WO 2007/054146

(56) Entgegenhaltungen:
- WO-A2-2004/038386
- DE-A1- 19 831 946

## Beschreibung

Die Erfindung bezieht sich auf eine Vorrichtung zum Ermitteln der Filtrierbarkeit von Fluiden, insbesondere von Getriebeölen, mit einer durch eine Probemenge des Fluides durchströmbaren Prüf-Filtereinrichtung.

Für die Zustandsüberwachung und Zustandserhaltung von technischen Anlagen, bei denen Fluide als Systemkomponenten beteiligt sind, ist es unerlässlich sicherzustellen, dass die Fluide, beispielsweise wenn sie als Schmierstoffe eingesetzt sind, im Laufe des Betriebes die erforderlichen Gebrauchseigenschaften beibehalten. So ist es beispielsweise bei Getriebeölen, die beim Durchlauf durch Systeme der Umlaufschmierung durch Kühl- und Filtereinrichtungen hindurch geführt werden, erforderlich, eine Prüfung der Filtrierbarkeit der betreffenden Öle durchzuführen. In üblicher Weise wird zu diesem Zweck eine Probemenge des Öles durch eine Prüf-Filtereinrichtung hindurch geführt, vorzugsweise in der Weise, dass eine Mehrzahl von hintereinander folgenden Durchgängen des Öles durch die Filtermedien vorgenommen wird, dass also eine Anzahl von Filtrationszyklen durchgeführt wird. Auf diese Weise lassen sich unter anderem die Verträglichkeiten zwischen den gegebenen Ölsorten und den Filtermedien ermitteln. Weiter können dergestalt getestet werden die Filtergängigkeit sowie die Materialverträglichkeit zwischen Öl und Filter, insbesondere hinsichtlich Additiven, wie beispielsweise Anti-Schaum und Verschleißschutz (EP/AW). Ferner kann der Einfluß der Filtration auf die Schaumbildung überprüft werden.

Wie sich jedoch gezeigt hat, besteht die Gefahr, dass bei Ölen, die mit einem Anti-Schaum Additivpaket versehen sind, das im Betrieb keine ausreichende Stabilität aufweist, es nach längerer Filtrationsdauer zu einer Abreicherung der Anti-Schaumadditive kommt, so dass ein verstärktes, fallweise schädliches Aufschäumen des Öles eintritt. Daher ist es erforderlich, das Öl nach Ablauf einer entsprechenden Anzahl von Filtrationszyklen auf das Schäumverhalten hin zu überprüfen.

Dokument WO 2004/038386 A2 offenbart eine Vorrichtung zur Bestimung der Oberflächenschaumbildung von Gretriebeölen.

Im Hinblick hierauf stellt sich die Erfindung die Aufgabe, eine Vorrichtung zu schaffen, die im Rahmen der Ermittlung der Filtrierbarkeit von Fluiden, insbesondere von Getriebeölen, die Möglichkeit der Überprüfung von deren Aufschäumverhalten bietet.

Erfindungsgemäß ist diese Aufgabe durch eine Vorrichtung gelöst, die die Merkmale des Anspruches 1 in seiner Gesamtheit aufweist.

Während bislang bei den Prüfungen der Filtrierbarkeit die Ermittlung des Schäumverhaltens gesondert von den eigentlichen Filtrationszyklen durchgeführt wurde, indem aus dem Filtrationsprüfstand nach Durchführung der entsprechenden Anzahl von Filtrationszyklen eine Entnahme von Nebenproben vorgenommen wurde, die gesondert auf das Schäumverhalten untersucht wurden, ist erfindungsgemäß der Schaumtest direkt in den Filtrationsprüfstand integriert. Eine Reihe von Nachteilen ist dadurch behoben. Während sich früher das Fluidvolumen im Filtrationsprüfstand wegen der Entnahme einer vergleichsweise großen Menge der Nebenprobe änderte, was eine Änderung der Prüfbedingungen zur Probe hatte, bleiben Volumen und Prüfbedingungen somit nunmehr gleich. Eine Ausnahme hiervon stellt die geringfügige Probenentnahme für optionale Laboruntersuchungen dar, wie z.B. besondere Formen der Emissionsspektroskopie. Dadurch entsteht auch keinerlei Zeitverlust für das Entnehmen der Nebenprobe, den Transport, den Schaumtest und den Rücktransport der Nebenprobe zum Filtrationsprüfstand. Zudem besteht keine Gefahr, dass äußere Einflüsse (Verschmutzung, Wasser und dergleichen) bei der Handhabung der Nebenprobe eine Fehlerquelle für die sensiblen Prüfabläufe darstellen könnten.

Für die erfindungsgemäße Vorrichtung ist ein Schaumtester besonders gut geeignet, der ein die Probemenge des Fluides aufnehmendes Prüfgehäuse aufweist, in dem eine eine Vermischung des Fluides mit Luft bewirkende Schaumbildnereinrichtung enthalten ist. Derartige Schaumtester sind beispielsweise unter der Handelsbezeichnung Flender-Schaumtest (A. Friedr. Flender GmbH) zu erhalten.

Vorzugsweise weist das Leitungssystem des Umwälzkreislaufes eine von der Filtereinrichtung zum Prüfgehäuse des Schaumtesters führende Rücklaufleitung auf, die das Fluid unterhalb des Flüssigkeitsspiegels der Probemenge in das Prüfgehäuse einspeist. Dadurch ist vermieden, dass in den Betriebsphasen zwischen den eigentlichen Schaumtests, also in den Zeiträumen des Stillstandes des als Schaumbildner dienenden Zahnradpaares, ein Aufschäumen durch den Rückstrom des umgewälzten, aus der Filtereinrichtung austretenden Öles bewirkt wird.

Vorzugsweise ist als Prüf-Filtereinrichtung eine Membran-Filtereinrichtung mit einer oder mehreren Filterlagen vorgesehen, wobei das Filtermedium vorzugsweise dem Filtermaterial entspricht, wie es bei der technischen Einrichtung (beispielsweise einem Getriebe einer Windkraftanlage) benutzt wird, deren Zustand mittels der erfindungsgemäßen Vorrichtung überwacht werden soll. Alternativ können sterngefaltete Filterelemente oder Beutelfilter sowie vergleichbare Filteranordnungen zur Anwendung kommen.

Nachstehend ist die Erfindung anhand eines in der Zeichnung dargestellten Ausführungsbeispieles im einzelnen erläutert. Es zeigen:
- Fig. 1 eine schematische Darstellung zur Verdeutlichung eines Ausführungsbeispieles der erfindungsgemäßen Vorrichtung, wobei ein Flender-Schaumtester in Seitenansicht gezeigt und ein Umwälzkreislauf für ein auf Filtrierbarkeit zu prüfendes Fluid als Blockschaltbild dargestellt sind, und
- Fig. 2 und 3 Diagramme, in denen die Ergebnisse der mit der erfindungsgemäßen Vorrichtung durchgeführten Prüfung zweier Getriebeöle dargestellt sind.

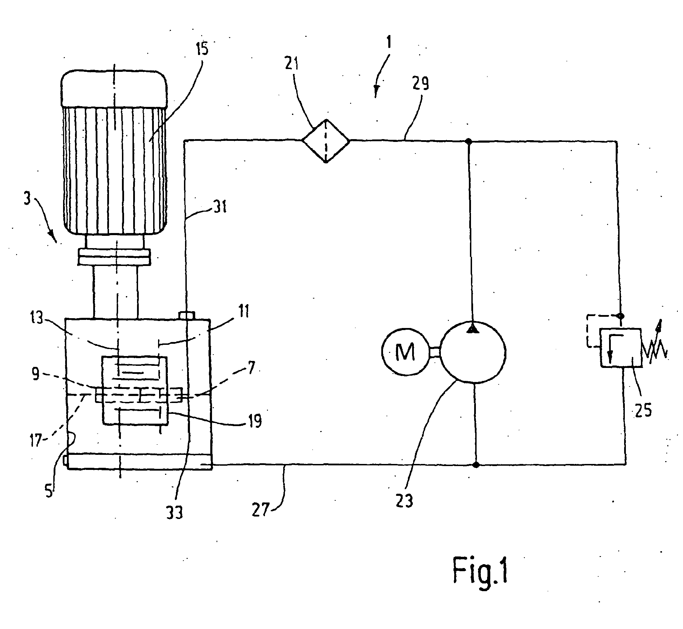

Bei dem in Fig. 1 gezeigten Ausführungsbeispiel der erfindungsgemäßen Vorrichtung ist ein Umwälzkreislauf für ein zu untersuchendes Fluid als ganzes mit 1 bezeichnet. Nachstehend ist die Wirkungsweise der Vorrichtung anhand des Beispieles der Untersuchung eines Fluides in Form eines Getriebeöles für das Getriebe einer Windkraftanlage erläutert. In den Umwälzkreislauf 1 ist ein handelsüblicher Flender-Schaumtester 3 integriert, der ein Prüfgehäuse 5 aufweist, dessen Innenraum für die Aufnahme einer gewünschten Probenmenge des zu prüfenden Öles, beim vorliegenden Beispiel ist dies ein Liter, bemessen ist. Im Prüfgehäuse befindet sich als Schaumbildnereinrichtung ein Zahnradpaar mit miteinander kämmenden Zahnrändern 7 und 9 auf vertikalen Achsen 11 bzw. 13, wobei die Achse 13 eine durch einen Elektromotor 15 getriebene Antriebswelle für das Zahnrad 9 bildet.

Wie durch ein Sichtfenster 19 im Prüfgehäuse 5 erkennbar ist, befinden sich die Zahnräder 7 und 9 auf einem Höhenniveau, in dem sie zur Hälfte in den mit 17 bezeichneten Fluidspiegel eintauchen, so dass die Drehbewegung der Zahnräder 7 und 9 eine Vermischung von Öl und Luft bewirkt.

Als weitere Bestandteile weist der Umwälzkreislauf 1, in den der Schaumtester 3 integriert ist, eine Prüf-Filtereinrichtung 21, eine Umwälzpumpe 23 in Form eine elektromotorisch angetriebenen Zahnradpumpe sowie ein Druckbegrenzungsventil 25 auf, das der Umwälzpumpe 23 nebengeschaltet ist. Die Saugseite der Umwälzpumpe 23 ist über eine Leitung 27 mit dem Innenraum des Prüfgehäuses 5 des Schaumtesters 3 in der Weise verbunden, dass die Umwälzpumpe 23 Fluid aus dem Bogenbereich des Prüfgehäuses 5 entnimmt. Die Druckseite der Pumpe 23 ist über eine Druckleitung 29 mit der Eintrittsseite der Filtereinrichtung 21 verbunden, deren Ausgangsseite über eine Rücklaufleitung 31 wiederum mit dem Prüfgehäuse 5 des Schaumtesters 3 verbunden ist, wobei die Mündung 33 der Rücklaufleitung 31, wie aus Fig. 1 ersichtlich, unterhalb des Fluidspiegels 17 im Prüfgehäuse 5 gelegen ist. Anstelle der genannten Zahnradpumpe können auch andere Antriebsvorrichtungen für das Fluid eingesetzt sein.

Zur Prüfung der Filtrierbarkeit eines betreffenden Fluides wird das Prüfgehäuse 5 mit einer Probemenge des zu testenden Fluides gefüllt, beispielsweise mit Getriebeöl in einer solchen Menge (z. B. etwa ein Liter), dass nach Füllen des Umwälzkreislaufes 1 (Leitungen, Pumpe, Filter) die Zahnräder 7 und 9 zur Hälfte in den Fluidspiegel 7 eingetaucht sind. Vor der Durchführung einer entsprechenden Anzahl von Filtrationszyklen, d. h. vor dem Umwälzen des eingefüllten "Frischöles", wird nun durch Betrieb des Schaumtesters 3 das ursprüngliche Schäumverhalten ermittelt, wobei an einer Skaleneinteilung am Sichtfenster 19 die prozentuale Volumenänderung ermittelt wird, die sich nach einer betriebsspezifischen Wartezeit nach Durchführen des Schaumtests einstellt.

Sodann wird eine gegebene Anzahl von Filtrationszyklen durch Betreiben der Umwälzpumpe 23 im Umwälzkreislauf durchgeführt, wobei unter Zyklus hier der Zeitbedarf zu verstehen ist, den das Prüföl benötigt, um - statistisch gesehen - einmal durch die Prüf-Filtereinrichtung 21 gefördert zu werden. Die Zyklendauer kann als Quotient aus Fluidvolumen und Volumenstrom berechnet werden.

Nach Durchführen der gewünschten Anzahl von Filtrationszyklen, beispielsweise 100 Zyklen, wird wiederum der Umwälzvorgang unterbrochen und ein Schaumtest durchgeführt, um die Veränderung des Schäumverhaltens zu ermitteln. Um festzustellen, wie sich das Schäumverhalten nach einer noch längeren Betriebszeit, d. h. nach Durchführen einer großen Anzahl von Filtrationsvorgängen ändert, wird sodann eine größere Anzahl von Filtrationszyklen durchgeführt, wonach der Schaumtester 3 erneut in Betrieb genommen wird, um das Schäumverhalten zu ermitteln, das das Prüföl nach längerer Betriebszeit zeigt.

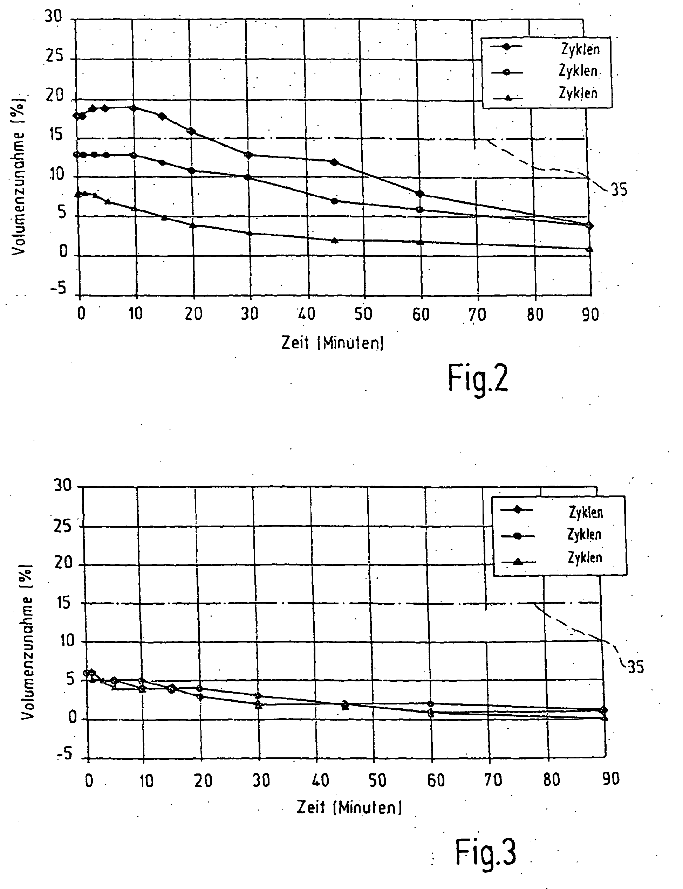

Fig. 2 zeigt die bei der Prüfung eines Getriebeöles ermittelten Testergebnisse anhand der durch Aufschäumen des Prüföles bewirkten, prozentualen Volumenzunahme. Je nach Öltyp oder Ölkategorie ist beispielsweise eine Volumenzunahme von 15 % als höchster zulässiger Grenzwert anzusehen.

Dieser Grenzwert ist in Fig. 2 durch eine strichpunktierte Linie 35 verdeutlicht. Wie in Fig. 2 durch die untere Kurve verdeutlicht ist, hat das Prüföl vor Durchführen von Filtrationszyklen ein anfängliches Schäumverhalten, das weit unterhalb des mit der Linie 35 angegebenen Grenzwertes liegt. Wie die mittlere Kurve zeigt, ist das Fremdverhalten auch nach Durchführen einer Vielzahl an Zyklen noch annehmbar. Dieses Prüföl hat jedoch bei längerer Betriebsdauer, siehe die obere Kurve, ein unbefriedigendes Schäumverhalten, entsprechend einer Volumenzunahme von mehr als 15 %. Die Prüfung dieser Ölsorte hat somit ergeben, dass das Prüföl ein nicht ausreichend stabiles Anti-Schaum Additivpaket enthält. Eine weitere Einflußgröße kann auch über eine Temperierung vorgegeben werden.

Demgegenüber zeigt Fig. 3 die Prüfungsergebnisse für eine Ölsorte, bei der die die Schaumbildung unterdrückenden Additive ein einwandfreies Betriebsverhalten zeigen. Sämtliche Kurven (für Versuchsbeginn, für wenige Zyklen und für sehr viele Zyklen) erstrecken sich weit unterhalb des mit der Linie 35 angegebenen Grenzwertes der 15%-igen Volumenzunahme durch Aufschäumen. Wie durch die jeweils obere Kurve für eine Vielzahl an Zyklen gezeigt, hat diese Ölsorte auch bei längerer Betriebsdauer ein gutes Betriebsverhalten, d. h. die Filtrierbarkeit wird nicht dadurch in Frage gestellt, dass sich Antischaumadditive an der Oberfläche des Filtermaterials absetzen, was eine Abreicherung dieser Additive und damit eine übermässige Schaumbildung zur Folge hätte. Die jeweils in den Fig. 2 und 3 dargestellten Zyklen betreffen bezogen auf das eingesetzte Dreieckssymbol den Versuchsbeginn, betreffend das Vierecksymbol das Versuchsende, und bezogen auf die kreis- oder punktförmige Angabe einen Zeitpunkt zwischen Versuchsbeginn und Versuchsende.

Die Filtereinrichtung 21 kann vorzugsweise Rundfilterelemente in einem Druckfilterhalter aufweisen. Vorzugsweise finden hierbei Filterronden aus dem gleichen Filtermedium Verwendung, wie es in der betreffenden technischen Anlage benutzt wird, deren Zustand jeweils überwacht werden soll. Anstelle der genannten Rundfilterelemente können auch gefaltete Filtermedien, Beutelfilter od. dgl. eingesetzt werden. Ferner bietet es sich an, den Schaumtester oder das Schaumprüfgerät als Ölbehälter für die Gesamtvorrichtung zu verwenden. Mit der erfindungsgemäßen Vorrichtung ist ein gut beherrschbares Meßverfahren vorgegeben, um eine Aussage über die Filtrierbarkeit von Medien zu treffen.

## Patentansprüche

1. Vorrichtung zum Ermitteln der Filtrierbarkeit von Fluiden, insbesondere von Getriebeölen, mit einer durch eine Probemenge des Fluides durchströmbaren Prüf-Filtereinrichtung (21) und einem einen Umwälzkreislauf (1) für das Fluid bildenden Leitungssystem (27, 29, 31), das einen die Probemenge des Fluides aufnehmenden Schaumtester (3), die Filtereinrichtung (21) und eine Umwälzpumpe (23) enthält, die Fluid aus der im Schaumtester (3) befindlichen Probemenge entnimmt und durch die Filtereinrichtung (21) hindurch und zum Schaumtester (3) zurück fördert.

2. Vorrichtung nach Anspruch 1, **dadurch gekennzeichnet, dass** der Schaumtester (3) ein die Probemenge des Fluides aufnehmendes Prüfgehäuse (5) aufweist, in dem eine eine Vermischung des Fluides mit Luft bewirkende Schaumbildnereinrichtung (7, 9) enthalten ist.

3. Vorrichtung nach Anspruch 2, **dadurch gekennzeichnet, dass** als Schaumbildnereinrichtung ein in den Flüssigkeitspegel (17) des Fluides teilweise eintauchendes, antreibbares, kämmendes Zahnradpaar (7, 9) vorgesehen ist.

4. Vorrichtung nach Anspruch 3, **dadurch gekennzeichnet, dass** das Leitungssystem des Umwälzkreislaufes (1) eine von der Filtereinrichtung (21) zum Prüfgehäuse (5) des Schaumtesters (3) führende Rücklaufleitung (31) aufweist, die das Fluid unterhalb des Flüssigkeitsspiegels (17) der Probemenge in das Prüfgehäuse (5) einspeist.

5. Vorrichtung nach einem der Ansprüche 1 bis 4, **dadurch gekennzeichnet, dass** als Prüf-Filtereinrichtung eine Membran-Filtereinrichtung (21) mit einer oder mehreren Filterlagen vorgesehen ist.

6. Vorrichtung nach Anspruch 5, **dadurch gekennzeichnet, dass** die Filtereinrichtung (21) Filterronden in einem Druckfilterhalter enthält.

## Claims

1. An apparatus for determining the filterabiltiy of fluids, in particular of transmission oils, having a test filter means (21) through which a sample amount of the fluid can flow, and a line system (27, 29, 31) forming a circulation circuit (1) for the fluid and which contains a foam tester (3) which holds the sample amount of the fluid, the filter means (21) and a circulation pump (23) which removes fluid from the sample amount located in the foam tester (3) and conveys it through the filter means (21) and back to the foam tester (3).

2. The apparatus according to Claim 1, **characterised in that** the foam tester (3) has a test housing (5) which holds the sample amountof fluid in which a foamer means (7, 9) which causes mixing of the fluid with air is contained.

3. The apparatus according to Claim 2, **characterised in that** a meshing gear pair (7; 9) which can be driven and which is partially immersed in the liquid level (17) of the fluid is provided as a foamer means.

4. The apparatus according to Claim 4, **characterised in that** the line system of the circulation circuit (1) has a return line (31) which leads from the filter means (21) to the test housing (5) of the foam tester (3) and which feeds the fluid below the liquid level (17) of the sample amount into the test housing (5).

5. The apparatus according to any of Claims 1 to 4, **characterised in that** a membrane filter means (21) with one or more filter layers is provided as the test filter means.

6. The apparatus according to Claim 5, **characterised in that** the filter means (21) contains filter rounds in a pressure filter holder.

## Revendications

1. Dispositif de détermination de l'aptitude à la filtration de fluides, notamment d'huiles de transmission, comprenant un dispositif (21) de filtration de contrôle, dans lequel peut passer une quantité échantillon du fluide, et un système (27, 29, 31) de canalisation formant un circuit (1) de recirculation du fluide, qui contient un testeur (3) de mousse recevant la quantité échantillon du fluide, le dispositif (21) de filtration et une pompe (23) de recirculation, qui prélève du fluide de la quantité échantillon se trouvant dans le testeur (3) de mousse et le retourne en passant à travers le dispositif (21) de filtration au testeur (3) de mousse.

2. Dispositif suivant la revendication 1, **caractérisé en ce que** le testeur (3) de mousse comporte un carter (5) de contrôle, qui reçoit la quantité échantillon du fluide, et dans lequel est contenu un dispositif (7, 9) de formation de mousse provoquant un mélange du fluide à de l'air.

3. Dispositif suivant la revendication 2, **caractérisé en ce qu'**il est prévu comme dispositif de formation de mousse une paire (7, 9) de roues dentées, qui engrènent, qui peuvent être entraînées et qui sont immergées en partie dans le niveau (17) de liquide du fluide.

4. Dispositif suivant la revendication 3, **caractérisé en ce que** le système de canalisation du circuit (1) de recirculation comporte un conduit (31) de retour allant du dispositif (21) de filtration au carter (5) de contrôle du testeur (3) de mousse et injectant le fluide dans le carter (5) de contrôle en dessous du niveau (17) de liquide de la quantité échantillon.

5. Dispositif suivant l'une des revendications 1 à 4, **caractérisé en ce qu'**il est prévu comme dispositif de filtration de contrôle un dispositif (21) de filtration en membrane ayant une ou plusieurs couches filtrantes.

6. Dispositif suivant la revendication 5, **caractérisé en ce que** le dispositif (21) de filtration comporte des rangs de filtres dans un porte-filtre sous pression.
